# EUROPEAN PATENT SPECIFICATION

(11) **EP 1 980 264 B1**
(45) Date of publication and mention of the grant of the patent: **15.07.2015**
(21) Application number: 06812136.7
(22) Date of filing: 23.10.2006
(51) Int. Cl.: A61K 38/44, A23K 1/165, A23L 1/30, A61P 1/00, A61P 1/04, A61P 1/12

(54) **PHARMACEUTICAL COMPOSITION, FOOD OR DRINK, OR FEED FOR INTESTINAL DISEASE**
PHARMAZEUTISCHE ZUSAMMENSETZUNG, NAHRUNGSMITTEL ODER GETRÄNK ODER FUTTERMITTEL FÜR DARMERKRANKUNGEN
COMPOSITION PHARMACEUTIQUE, ALIMENT, BOISSON OU NUTRIMENT POUR MALADIE INTESTINALE

(30) Priority: 20.01.2006 JP 2006011843
(43) Date of publication of application: 15.10.2008
(73) Proprietor: MORINAGA MILK INDUSTRY CO., LTD., Minato-ku, Tokyo 108-8384 (JP)
(72) Inventor: SHIN, Kouichirou, Kanagawa 228-8583 (JP)
(74) Representative: Hiebl, Inge Elisabeth
(86) International application number: PCT/JP2006/321076
(87) International publication number: WO 2007/083425

(56) References cited:
- EP-A1- 1 393 734
- WO-A1-92/13064
- WO-A1-96/13271
- WO-A1-2004/041004
- JP-A- 05 508 542
- JP-A- 2000 509 367
- JP-A- 2000 514 399
- JP-A- 2003 246 753
- US-A1- 2003 176 389
- VAN LEEUWEN P ET AL: "Effects of a lactoperoxidase system and lactoferrin, added to a milk replacer diet, on severity of diarrhoea, intestinal morphology and microbiology of digesta and faeces in young calves", JOURNAL OF ANIMAL PHYSIOLOGY AND ANIMAL NUTRITION, BLACKWELL, BERLIN, DE, vol. 83, no. 1, 1 February 2000 (2000-02-01), pages 15-23, XP009159208, ISSN: 0931-2439, DOI: 10.1046/J.1439-0396.2000.00250.X [retrieved on 2008-10-09]
- DATABASE MEDLINE [Online] US NATIONAL LIBRARY OF MEDICINE (NLM), BETHESDA, MD, US; January 1998 (1998-01), HUSAIN A ET AL: "Nutritional issues and therapy in inflammatory bowel disease.", XP9159212, Database accession no. NLM9547853 & HUSAIN A ET AL: "Nutritional issues and therapy in inflammatory bowel disease.", SEMINARS IN GASTROINTESTINAL DISEASE JAN 1998 LNKD- PUBMED:9547853, vol. 9, no. 1, January 1998 (1998-01), pages 21-30, ISSN: 1049-5118
- STREUTKER C.J. ET AL.: 'Detection of Species-Specific Helicobacter Ribosomal DNA in Intestinal Biopsy of Patients with Population-Based Cohort of Patients with Ulcerative Colitis' JOURNAL OF CLINICAL MICROBIOLOGY vol. 42, no. 2, 2004, pages 660 - 664, XP003010897
- DIONYSIUS D.A. ET AL.: 'Studies on the Lactoperoxidase System: Reaction Kinetics and Antibacterial Activity Using Two Methods for Hydrogen Peroxide Generation' JOURNAL OF APPLIED BACTERIOLOGY vol. 72, no. 2, 1992, pages 146 - 153, XP003010898
- DARFEUILLE-MICHAUD S.: 'Adherent-Invasive Escherichia coli: a Putative New E. coli Pathotype Associated With Crohn's Disease' INTERNATIONAL JOURNAL OF MEDICAL MICROBIOLOGY vol. 292, no. 3-4, 2002, pages 185 - 193, XP004959921
- MARTIN H.M. ET AL.: 'Enhanced Escherichia coli Adherence And Invasion in Crohn's Disease and Colon Cancer' GASTROENTEROLOGY vol. 127, no. 1, 2004, pages 80 - 93, XP005314279
- AXELSSON ET AL: "Experimental colitis induced by dextran sulphate sodium in mice: beneficial effects of sulphasalazine and olsalazine", ALIMENT PHARMACOLO THER, vol. 12, 1998, pages 925-934,
- MURAKAMI ET AL: "Supression of dextra sodium sulphate-induced colitis in mice by zerumbone, a subtropical ginger sesquiterpene, and nimesulide: separately and in combination", BIOCHEMICAL PHARMACOLOGY, vol. 66, 2003, pages 1253-1261,
- KANAUCHI ET AL: "Germinated barley foodstuff, a prebiotic product, ameliorates inflammation of colitis through modulation of the enteric environment", J GASTROENTEROLOGY, vol. 38, 2003, pages 134-141,
- ISHIKAWA HIDEKI ET AL: "Randomized controlled trial of the effect of bifidobacteria-fermented milk on ulcerative colitis", JOURNAL OF THE AMERICAN COLLEGE OF NUTRITION, AMERICAN COLLEGE OF NUTRION, WILMINGTON, NC, US, vol. 22, no. 1, 1 February 2003 (2003-02-01), pages 56-63, XP009180823, ISSN: 0731-5724
- KATO K ET AL: "Randomized placebo-controlled trial assessing the effect of bifidobacteria-fermented milk on active ulcerative colitis", ALIMENTARY PHARMACOLOGY & THERAPEUTICS, BLACKWELL SCIENTIFIC PUBLICATIONS LTD., CAMBRIDGE, GB, vol. 20, no. 10, 15 November 2004 (2004-11-15), pages 1133-1141, XP002536214, ISSN: 0269-2813, DOI: 10.1111/J.1365-2036.2004.02268.X

## Description

### Technical Field

The present invention relates to a pharmaceutical composition, a food or drink, or a feed containing lactoperoxidase as an active ingredient for prevention and/or treatment of intestinal disorders such as ulcerative colitis and Crohn's disease.

### Background Art

Ulcerative colitis and Crohn's disease are inflammatory bowel diseases whose causes remain unknown, and are also chronic refractory diseases with repeated remission and exacerbation. As for the causes of these diseases, it has been pointed out that, in addition to genetic causes and immune abnormalities, environmental factors such as meals may also be involved in these diseases (see, for example, Non-Patent Document 1), but as described above, the causes of these diseases remain unknown. Ulcerative colitis is an inflammatory disease in which continuous erosion or ulcer is formed in the mucous membrane of the large intestine, and it causes symptoms such as bloody stools, mucous and bloody stools, diarrhea, and stomachache and, in a serious case, further causes generalized symptoms such as fever, weight reduction, and anemia (see, for example, Non-Patent Document 2). Crohn's disease is a disease in which a discontinuous lesion such as inflammation or ulcer appears at any site in the digestive tract from the mouth to the anus, and it causes symptoms such as stomachache, diarrhea, and bloody stools, and in a serious case, further causes symptoms such as fever, melena, weight reduction, general malaise, and anemia (see, for example, Non-Patent Document 3).

It has been conventionally known that the prevalence of such inflammatory bowel diseases is high in Europe and the United States. In recent years, also in Japan, the number of patients of inflammatory bowel diseases has been rapidly increasing with changes in lifestyle habits such as dietary habits. However, as described above, the causes of inflammatory bowel diseases remain unknown, which is one reason for the fact that an fundamental causal treatment method for inflammatory bowel diseases has not yet been established. Therefore, the treatment strategy of inflammatory bowel diseases is aimed at not curing (i.e., complete cure) but early induction of remission (i.e., temporary improvement) and maintenance of remission for as long as possible. As pharmaceutical drugs for such symptomatic therapy, aminosalicylic acid formulations, adrenocortical steroid drugs, and immunosuppressive drugs are mainly used, but side-effects of these drugs are major clinical problems. For example, it has been reported that Salazosulfapyridine, often used as an aminosalicylic acid formulation, produces side-effects such as nausea, vomiting, anorexia, eruption, headache, hepatopathy, leucopenia, abnormal erythrocyte, proteinuria, and diarrhea. In the case of using an adrenocortical steroid drug, prednisolone is usually administered by oral, enema, or intravenous administration or in suppository form, but produces strong side-effects such as gastric ulcer and avascular necrosis of the femoral head caused by long-term use. However, these drugs have to be continuously administered because interruption of medication causes recurrence of symptoms.

On the other hand, irritable bowel syndrome is a disease in which an apparent organic lesion is not found in the small or large intestine but symptoms such as stomachache, diarrhea, mucous stools, constipation, and bloating occur by functional problems (see, for example, Non-Patent Document 4). According to a study in Japan, it has been reported that the prevalence of irritable bowel syndrome in Japan is as high as 15.5% that is at the same level as in Europe and the United States (see, for example, Non-Patent Document 4). The causes of irritable bowel syndrome also remain unknown, but it has been pointed out that in addition to psychologic stress and dysfunction of bowel movement, mild colitis which has not been completely cured may cause the above symptoms (see, for example, Non-Patent Document 5). As described above, since the causes of irritable bowel syndrome remain unknown, symptomatic therapy aimed at induction and maintenance of remission is performed also for irritable bowel syndrome. As pharmaceutical drugs for irritable bowel syndrome, intestinal function controlling drugs, laxatives, anticholinergic drugs, antidepressant drugs, and antianxiety drugs are mainly administered. Further, it has also been suggested that the symptoms of irritable bowel syndrome can be relieved by using a pharmaceutical drug used in symptomatic therapy of inflammatory bowel diseases (see, for example, Non-Patent Document 6). However, also in the case of irritable bowel disease, pharmaceutical drugs have to be continuously administered to induce and maintain remission although there is a major problem that the pharmaceutical drugs have side-effects.

Meanwhile, lactoperoxidase is an oxidoreductase having a molecular weight of about 80,000 and contained in mammalian milk, such as cow's milk, and secretions such as saliva, tears , and airway mucus (see, for example, Non-Patent Document 7), and it contains one heme as a coenzyme in a molecule (see, for example, Non-Patent Document 8). It has been known that lactoperoxidase oxidizes thiocyanic acid (SCN-), widely contained in tissues and body fluids of animals, in the presence of hydrogen peroxide to produce hypothiocyanic acid (OSCN-) that is an antibacterial substance (see, for example, Non-Patent Document 9). Based on such a system, pharmaceutical drugs having various effects have been developed using lactoperoxidase, thiocyanic acid, and hydrogen peroxide (or compounds which can supply hydrogen peroxide). For example, there have been disclosed techniques such as use of lactoperoxidase, a peroxide donor, and thiocyanate for producing a medicament for treating Helicobacter pylori infection (Patent Document 1), application of peroxidase to prevention and treatment (Patent Document 2), a method for producing a lactic acid bacteria fermented food containing a peroxidase-thiocyanic acid ion and/or halogeno ion-hydrogen peroxide system (Patent Document 3), a two enzyme dentifrice (Patent Document 4), a microbicidal composition (Patent Document 5), a lactic acid bacteria starter containing peroxidase, a fermented milk product, and a method for producing the same (Patent Document 6), and a urease-inactivating composition and a food or drink (Patent Document 7).

Further, it has also been reported that when lactoperoxidase was orally administered alone to mice infected with influenza virus, the severity of pneumonia, the number of inflammatory cells contained in a bronchoalveolar lavage fluid, and the concentration of Interleukin 6 in blood serum were suppressed (see, for example, Non-Patent Document 10). Further, there have also been disclosed techniques such as an intestinal flora improver and a food or drink (Patent Document 8), a protease inhibitor (Patent Document 9), an antiviral agent (Patent Document 10), and an Interleukin 6 production inhibitor (Patent Document 11). However, techniques relating to the effect of lactoperoxidase on intestinal disorders have not been reported at all until now.

Patent Document 1: Japanese Patent Application National Publication (Laid-Open) No. 2000-509367
Patent Document 2: Japanese Patent No. 3478821
Patent Document 3: Japanese Patent Application Laid-open No. 62-228224
Patent Document 4: Japanese Patent Application Publication No. 4-25924
Patent Document 5: Japanese Patent Application Laid-open No. 1-61427
Patent Document 6: Japanese Patent No. 3007686
Patent Document 7: Japanese Patent Application Laid-open No. 2002-238554
Patent Document 8: Japanese Patent Application Laid-open No. 2003-246753
Patent Document 9: Japanese Patent Application Laid-open No. 2005-104900
Patent Document 10: Japanese Patent Application Laid-open No. 2005-232133
Patent Document 11: pamphlet of International Patent Publication No. 2004/073733
Non-Patent Document 1: Japanese Journal of Clinical Medicine vol. 63, pp. 757-762,2005
Non-Patent Document 2: Japanese Journal of Clinical Medicine vol. 63, pp. 744-749,2005
Non-Patent Document 3: Diseases of the Colon and Rectum, vol. 43, pp. S85-S93, 2000, USA
Non-Patent Document 4: Internal Medicine, vol. 43, pp. 353-359,2004, Japan
Non-Patent Document 5: Gut, vol. 51, pp. i41-i44, 2002, UK
Non-Patent Document 6: Current Opinion in Gastroenterology, vol. 19, pp. 336-342,2003, USA
Non-Patent Document 7: American Journal of Respiratory and Critical Care Medicine, vol. 166, pp. S57-S61, 2002, USA
Non-Patent Document 8: British Journal of Nutrition, vol. 84, pp. S19-S25, 2000, UK
Non-Patent Document 9: American Journal of Respiratory Cell and Molecular Biology, vol. 20, pp. 642-644, 2000, USA
Non-Patent Document 10: Journal of Medical Microbiology, vol. 54, pp. 717-723, 2005, UK

Further relevant literature:
US 2003/176389 A1 (RAZ ET AL) 18 September 2003
EP 1 393 734 A1 (AJINOMOTO) 3 March 2004
AXELSSON ET AL: "Experimental colitis induced by dextran sulphate sodium in mice: beneficial effects of sulphasalazine and olsalazine", ALIMENT PHARMACOLO THER, vol. 12, 1998, pages 925-934
MURAKAMI ET AL: "Supression of dextra sodium sulphate-induced colitis in mice by zerumbone, a subtropical ginger sesquiterpene, and nimesulide: separately and in combination", BIOCHEMICAL PHARMACOLOGY, vol. 66, 2003, pages 1253-1261.
KANAUCHI ET AL: "Germinated barley foodstuff, a prebiotic product, ameliorates inflammation of colitis through modulation of the enteric environment", J GASTROENTEROLOGY, vol. 38, 2003, pages 134-141

### Disclosure of the Invention

### Problems to be solved by the Invention

Under the circumstances, there has been a demand for development of an agent for preventing or treating intestinal disorders such as ulcerative colitis and Crohn's disease, which is safe, produces no side-effects, and can be administered or ingested for a long time to induce and maintain remission.

In addition, there has also been a demand for a food or drink which is useful for prevention of intestinal disorders and/or improvement of symptoms of intestinal disorders (i.e., induction and maintenance of remission) and which can be continuously and habitually ingested in daily life as a health food, a functional food, a food for special dietary uses, a food with nutrient function claims, a food for specified health uses, or the like without the fear of side-effects.

### Means for solving the problems

In light of the above problems, the present inventors have extensively studied, and as a result have found for the first time that lactoperoxidase, being a milk protein, has the effect of preventing intestinal disorders such as ulcerative colitis and Crohn's diseases and relieving (improving) symptoms of these intestinal disorders, and thus completed the present invention.

It is therefore an object of the present invention to provide a pharmaceutical composition, a food or drink, or a feed containing lactoperoxidase as an active ingredient for prevention and/or treatment of intestinal disorders such as ulcerative colitis, Crohn's disease. The above object is achieved by the following means.

A first invention to achieve the above object is a pharmaceutical composition for prevention and/or treatment of an intestinal disorder containing lactoperoxidase as an active ingredient. According to a preferred aspect of the first invention, the intestinal disorder is at least one or more selected from among ulcerative colitis and Crohn's disease.

A second invention to achieve the above object is a food or drink for prevention and/or treatment of an intestinal disorder containing lactoperoxidase as an active ingredient. According to a preferred aspect of the second invention, the intestinal disorder is at least one or more selected from among ulcerative colitis and Crohn's disease, and the food or drink is provided as a health food, a functional food, a food for special dietary uses, a food with nutrient function claims, or a food for specified health uses.

A third invention to achieve the above object is use of lactoperoxidase for producing the food or drink according to the second invention.

A fourth invention to achieve the above object is a food additive for prevention and/or treatment of an intestinal disorder containing lactoperoxidase as an active ingredient. According to a preferred aspect of the fourth invention, the intestinal disorder is at least one or more selected from among ulcerative colitis and Crohn's disease.

A fifth invention to achieve the above object is a feed for prevention and/or treatment of an intestinal disorder containing lactoperoxidase as an active ingredient. According to a preferred aspect of the fifth invention, the intestinal disorder is at least one or more selected from among ulcerative colitis and Crohn's disease.

A sixth invention to achieve the above object is a method for prevention and/or treatment of an intestinal disorder of mammals by administrating a pharmaceutical composition and/or a feed containing lactoperoxidase as an active ingredient. According to a preferred aspect of the sixth invention the intestinal disorder is at least one or more selected from among ulcerative colitis and Crohn's disease.

The present invention provides the following (1) to (11).
(1) A pharmaceutical composition for prevention and/or treatment of an intestinal disorder containing lactoperoxidase as an active ingredient and a pharmaceutically acceptable carrier.
(2) The pharmaceutical composition according to the above (1), wherein the intestinal disorder is at least one or more selected from among ulcerative colitis and Crohn's disease.
(3) Use of lactoperoxidase for producing the pharmaceutical composition according to the above (1) or (2).
(4) A food or drink for prevention and/or treatment of an intestinal disorder containing lactoperoxidase as an active ingredient.
(5) The food or drink according to the above (4), wherein the intestinal disorder is at least one or more selected from among ulcerative colitis and Crohn's disease.
(6) Use of lactoperoxidase for producing the food or drink according to the above (4) or (5).
(7) The food or drink according to any one of the above (4) to (6), which is provided as a health food, a functional food, a food for special dietary uses, a food with nutrient function claims, or a food for specified health uses.
(8) A food additive for prevention and/or treatment of an intestinal disorder containing lactoperoxidase as an active ingredient.
(9) The food additive according to the above (8), wherein the intestinal disorder is at least one or more selected from among ulcerative colitis and Crohn's disease.
(10) A feed for prevention and/or treatment of an intestinal disorder containing lactoperoxidase as an active ingredient.
(11) The feed according to the above (10), wherein the intestinal disorder is at least one or more selected from among ulcerative colitis and Crohn's disease.
(12) A method for prevention and/or treatment of an intestinal disorder of mammals by administrating a pharmaceutical composition containing lactoperoxidase as an active ingredient and a pharmaceutically acceptable carrier.
(13) The method according to the above (12), wherein the intestinal disorder is at least one or more selected from among ulcerative colitis and Crohn's disease.

As for the lactoperoxidase mentioned above, one isolated from natural material can be used. Alternatively, peroxidase artificially synthesized may also be used. However, lactoperoxidase isolated from natural substance is preferably used.

### Effect of the Invention

The present invention produces the following effects.

(1) The pharmaceutical composition, food and drink, and food additive according to the present invention have the effect of preventing and/or treating intestinal disorders such as ulcerative colitis and Crohn's disease.
(2) The pharmaceutical composition, food and drink, and food additive according to the present invention are highly safe for humans and animals and therefore can be continuously and daily ingested for a long time without the fear of side-effects to prevent and/or treat intestinal disorders.
(3) Lactoperoxidase to be used in the present invention as an active ingredient can be stably produced in large quantity from a raw material such as milk or whey available as a biomaterial relatively cheaply, and therefore it is possible to provide a pharmaceutical composition, a food or drink, or a food additive for prevention and/or treatment of intestinal disorders at low price. This relieves the burden of expense, which makes it easy to continuously and daily ingest lactoperoxidase for a long time.

### Best Mode for Carrying Out the Invention

Hereinbelow, the present invention will be described in detail with reference to preferred embodiments of the present invention. The present invention is not limited to the following preferred embodiments, and various changes may be made without departing from the scope of the present invention. It is to be noted that in this specification, all percentages (%) are by mass unless otherwise specified.

In the present disclosure the effect of prevention and treatment of intestinal disorders refers to the effect of prevention and treatment of mainly inflammatory bowel diseases, especially symptoms of inflammatory bowel diseases, such as ulcerative colitis and Crohn's disease, and irritable bowel syndrome caused by mild inflammation which has not been completely cured. Further, the effect of treatment includes the effect of relieving (improving) symptoms of intestinal disorders and the effect of treating intestinal disorders, and particularly means the effect of inducing and maintaining remission. More specifically, daily administration or ingestion of lactoperoxidase used as an active ingredient makes it possible to obtain the effect of prevention and/or treatment of these intestinal disorders with little side effects.

Lactoperoxidase to be used in the present invention can be industrially produced by, for example, a method disclosed in Japanese Patent Application Laid-open No. H5-41981 entitled "Viable cell-containing liquid composition" in which lactoperoxidase is produced from unheated whey or skimmed milk in the usual manner (e.g., ion chromatography). Alternatively, if desired, commercially available naturally occurring lactoperoxidase (e.g., lactoperoxidase manufactured by Biopole) or recombinant lactoperoxidase [e.g., recombinant lactoperoxidase developed and purified by a method by Shin et al. (see Biochemical and Biophysical Research Communications, vol. 271, pp. 831-836, 2000) or commercially available recombinant lactoperoxidase] may also be used.

Lactoperoxidase to be used in the present invention is preferably derived from mammalian milk. In the case of producing a pharmaceutical composition or a food or drink for humans, lactoperoxidase to be used is preferably derived from milk of cows, sheep, or goats whose milk has been traditionally used for foods and drinks, and is particularly preferably derived from cow's milk. This is because such milk has been traditionally used for foods and drinks for humans for a long time and therefore its safety for humans is assured at a very high degree.

Particularly, unheated whey derived from cow's milk is particularly preferred as a raw material of lactoperoxidase to be used in the present invention because it can be stably obtained in large quantity as a by-product during production of milk products.

Lactoperoxidase contained in the pharmaceutical composition, food or drink, or feed according to the present invention as an active ingredient is a natural product derived from milk, which is highly safe when ingested, and is contained in foods such as cow's milk, therefore it is daily ingested, possesses no toxicity, and produces little side effects even when continuously ingested for a long time. Therefore, lactoperoxidase can be appropriately administered by various administration methods such as oral administration, and can also be processed into tablets, capsules, troches, syrups, granules, powders or the like by known methods. Lactoperoxidase can also be contained in a food as an active ingredient to provide a functional food, having the effect of prevention and/or treatment of intestinal disorders, as one aspect for prevention and/or treatment of intestinal disorders.

The dose of lactoperoxidase contained in the pharmaceutical composition, food or drink, or feed according to the present invention as an active ingredient varies depending on dosage form, symptoms, age, and body weight, and the like but at least 1 mg/kg body weight/day is preferably orally administered, and 3 to 300 mg/kg body weight/day is particularly preferably administered to effectively prevent and/or treat intestinal disorders.

The pharmaceutical composition according to the present invention can be produced by, for example, preparing a formulation using lactoperoxidase and one or more desired pharmaceutically acceptable additives such as excipients. In this case, the amount of lactoperoxidase contained in the formulation is usually 0.001 to 10% by mass, preferably 0.01 to 10% by mass. Examples of the additives to be used for preparing the formulation include excipients, binders, disintegrants, lubricants, stabilizers, flavoring agents, diluents, and solvents for injections.

Examples of the excipients include: sugar derivatives such as lactose, sucrose, glucose, mannite, and sorbitol; starch derivatives such as maize starch, potato starch, α-starch, dextrin, and carboxymethyl starch; cellulose derivatives such as crystalline cellulose, hydroxypropyl cellulose, hydroxypropylmethyl cellulose, carboxymethyl cellulose, and carboxymethylcellulose calcium; gum arabic; dextran; pullulan; silicate derivatives such as light anhydrous silicic acid, synthetic aluminum silicate, and magnesium metasilicate aluminate; phosphate derivatives such as calcium phosphate; carbonate derivatives such as calcium carbonate; and sulfate derivatives such as calcium sulfate. Examples of the binders include in addition to the above-mentioned excipients, gelatin, polyvinyl pyrrolidone, and macrogol. Examples of the disintegrants include in addition to the above-mentioned excipients, chemically-modified starch or cellulose derivatives such as croscarmellose sodium, carboxymethylstarch sodium, and crosslinked polyvinyl pyrrolidone. Examples of the lubricants include talc; stearic acid; metal stearates such as calcium stearate and magnesium stearate; colloidal silica; waxes such as bee gum and spermaceti wax; boric acid; glycol; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acids such as anhydrous silicic acid and silicic acid hydrate; and starch derivatives. Examples of the stabilizers include p-oxybenzoates such as methylparaben and propylparaben; alcohols such as chlorobutanol, benzyl alcohol, and phenylethyl alcohol; benzalkonium chloride; acetic anhydride; and sorbic acid. Examples of the flavoring agents include sweeteners, acidulants, and perfumes. Examples of the solvents for injections include water, ethanol, and glycerin.

Examples of the administration routes of the pharmaceutical composition according to the present invention include oral administration and non-oral administration such as enteral administration. Examples of the dosage form of the pharmaceutical composition according to the present invention include sprays, capsules, tablets, granules, syrups, emulsions, suppositories, injections, ointments, and tapes. The pharmaceutical composition according to the present invention may also be administered by mixing with a food or drink or a feed.

A food or drink containing the pharmaceutical composition according to the present invention means a food or drink for prevention and/or treatment of intestinal disorders containing lactoperoxidase as an active ingredient, and examples of such a food or drink include those which can be daily ingested.

The food or drink containing the pharmaceutical composition according to the present invention can be produced by, for example, mixing lactoperoxidase with sugar (e.g., dextrin or starch), protein (e.g., gelatin, soybean protein, or maize protein), amino acid (e.g., alanine, glutamine, or isoleucine), polysaccharide (e.g., cellulose or gum arabic), or fat or oil (e.g., soybean oil or medium chain fatty acid triglyceride). Examples of the form of such a food or drink composition include drinks such as cold beverages, carbonated drinks, nutritional drinks, fruit drinks, and lactic acid drinks (including concentrated or undiluted liquids thereof and instant powders thereof); frozen desserts such as ice creams, ice sorbets, and ice shavings; noodles such as Japanese buckwheat noodles, Japanese wheat noodles, bean-starch noodles, paste wrappings for Chinese stuffed dumplings, paste wrappings for Chinese steamed dumplings, Chinese noodles, and instant noodles; confectionaries such as hard candies, chewing gums, candies, gums, chocolates, tablets, snack foods, biscuits, jellies, jams, creams, and baked goods; fishery and livestock processed foods such as steamed fish pastes, hams, and sausages; milk products such as processed milk and fermented milk; fats and oils and fat and oil processed foods such as cooking oils, frying oils, margarine, mayonnaise, shortening, whipped cream, and dressings; seasonings such as sauces and bastings; soups; stews; salads; prepared meals; pickles; breads; enteral nutritional foods; and functional foods.

A feed containing the pharmaceutical composition according to the present invention means a feed for prevention and/or treatment of intestinal disorders containing lactoperoxidase as an active ingredient, and examples of such a feed include those which can be daily fed.

The feed containing the pharmaceutical composition according to the present invention can be produced by, for example, mixing lactoperoxidase with grain (e.g., maize, wheat, barley, rye, or milo); vegetable oil cake (e.g., soybean oil cake, rape oil cake, palm oil cake, or linseed oil cake); bran (e.g., wheat bran, oat bran, rice bran, or defatted rice bran); by-product meal (e.g., corn gluten meal or corn germ meal); animal feed (e.g., fish meal, skimmed milk powder, whey, yellow grease, or tallow); yeast (e.g., torula yeast or beer yeast); mineral feed (e.g., tribasic calcium phosphate or calcium carbonate); oil or fat; single amino acid; or sugar. Examples of the form of such a feed include pet foods, feeds for livestock, and feeds for cultured fish.

The pharmaceutical composition, food or drink, or feed according to the present invention can be used alone or together with another pharmaceutical composition, food or drink, or feed effective against intestinal disorders. By using the pharmaceutical composition, food or drink, or feed according to the present invention together with another pharmaceutical composition, food or drink, or feed effective against intestinal disorders, it is possible to enhance the effect of prevention and/or treatment of intestinal disorders. Another pharmaceutical composition, food or drink, or feed to be used together with the pharmaceutical composition, food or drink, or feed according to the present invention may be contained in the pharmaceutical composition, food or drink, or feed according to the present invention as an active ingredient, or may be commercialized as another pharmaceutical drug or food or drink without being contained in the pharmaceutical composition, food or drink, or feed according to the present invention.

The food or drink according to the present invention can be used for various purposes utilizing its effect of prevention and/or treatment of intestinal disorders.

The food or drink according to the present invention is preferably sold as a food or drink with an indication that its intended use is to improve intestinal disorders, for example, "a food or drink having the effect of improving intestinal disorders with an indication 'For improvement of intestinal disorders"', "a food or drink containing lactoperoxidase with an indication 'For improvement of intestinal disorders"', or "a food or drink containing lactoperoxidase with an indication 'For prevention of intestinal disorders"'.

It goes without saying that a phrase to be used for the above indication is not limited to "For improvement of intestinal disorders" or "For prevention of intestinal disorders", and other phrases also fall within the scope of the present invention as long as they express the effect of prevention or treatment of intestinal disorders. As such phrases, for example, those based on various intended uses allowing customers to recognize the effect of preventing and/or improving intestinal disorders are also possible.

The action of displaying the "indication" described above includes all actions for informing customers about the intended use described above, and any actions fall within the scope of the "indication" according to the present invention irrespective of the purpose, content, target object, medium, or the like of the indication, as long as they can remind the customers of the intended use described above. However, the indication is preferably expressed so that customers can directly recognize the intended use described above. Specific examples of such actions include: actions of displaying the intended use described above on products according to the food or drink of the present invention or on packages of the products; actions of assigning, delivering, displaying for the purpose of assigning or delivering, or importing products with an indication of the intended use described above or packages of the products; and actions of displaying or distributing advertisements, price lists, or business papers relating to the products with the intended use described above, or providing information including those as contents with the intended use described above by an electromagnetic method (e.g., by the Internet).

On the other hand, contents of the indication are preferably approved by the government or the like (e.g., an indication in a form approved based on any one of various systems established by the government), and such contents of the indication are preferably displayed on packages, containers, advertisement materials used on sales sites, such as catalogues, brochures, or POPs, other documents, or the like.

Examples of the indication further include indications as health food, functional food, enteral nutritional food, food for special dietary uses, health-promoting food, food for specified health uses, food with nutrient functional claims, and quasi-drug and indications approved by the Ministry of Health, Labour, and Welfare such as indications approved based on the system for food for specified health uses and on other systems similar to it. Examples of the latter include indications as food for specified health uses and indications as qualified food for specified health uses, indications that body structures and functions are influenced, and indications of reduction of disease risk claims, and more specifically, typical examples thereof include indications as food for specified health uses (especially, indications of health uses) provided in the enforcement regulations of Health Promotion Law (Japan Ministry of Health, Labour, and Welfare, Ministerial ordinance No. 86, April 30, 2003) and indications similar to them.

In the present invention, the effect of lactoperoxidase on prevention and/or treatment of intestinal disorders was evaluated by carrying out a test using animal colitis models based on induction of colitis by administration of dextran sulfate sodium described in Gastroenterology, vol. 109, pp. 1344-1367, 1995, USA or Laboratory Investigation, vol. 69, pp.238-249, 1993, USA. More specifically, colitis was induced by feeding drinking water containing dextran sulfate sodium to mice in the usual manner, and then the effect of lactoperoxidase on symptoms of colitis (e.g., weight loss-inhibiting effect, colon shortening-inhibiting effect, and colitis-inhibiting effect) was checked using the mice.

Hereinbelow, the present invention will be described in more detail with reference to the following examples, but the present invention is not limited to these examples.

### Example 1

### (Production of Lactoperoxidase)

10,000 kg of unheated whey was passed through a column filled with 100 L of CM-Sephadex C-50 (manufactured by Amersham Biosciences). Then, the sephadex gel was washed with 1,000 kg of water, and then 200 L of 20 mM sodium phosphate buffer (pH 7.0) containing 0.3 M sodium chloride was passed through the column to elute lactoperoxidase adsorbed to the sephadex resin. The thus obtained eluate was concentrated and deionized using an ultrafiltration membrane module having a molecular weight cut off of 10,000 (manufactured by Asahi Kasei Corporation). To about 10 L of thus obtained concentrated liquid, 30 L of deionized water was added, and then the liquid was passed through a column filled with 20 L of SP-Sepharose FF (manufactured by Amersham Bioscience). Then, the sepharose gel was washed with 20 mM sodium phosphate buffer (pH 6.4) containing 0.15 M sodium chloride, and then lactoperoxidase was eluted using 20 mM sodium phosphate buffer (pH 6.4) containing 0.3 M sodium chloride. The thus obtained eluate was concentrated and deionized using an ultrafiltration membrane module having a molecular weight cut off of 10,000 (manufactured by Asahi Kasei Corporation). Then, the concentrated liquid was aseptically filtered using a microfiltration membrane module having an average pore size of 0.2 µm (manufactured by Asahi Kasei Corporation) and freeze dried to obtain about 150 g of an aseptic lactoperoxidase powder.

The lactoperoxidase preparation was subjected to electrophoresis using an SDS-polyacrylamide gel having an acrylamide concentration of 10 to 20%, stained with Coomasie brilliant blue R250 (manufactured by Sigma), and analyzed with an image processor (manufactured by ATTO Corporation). As a result, it was found that the purity of the lactoperoxidase was about 97%.

### Example 2

### (Production of Lactoperoxidase Tablet)

150 g of the lactoperoxidase obtained in the Example 1, 100 g of a lactulose powder (manufactured by Morinaga Milk Industry Co., Ltd.), 635 g of malt dextrin (manufactured by Matsutani Chemical Industry Co., Ltd.), 85 g of skimmed milk (manufactured by Morinaga Milk Industry Co., Ltd.), 1 g of a stevia sweetener (manufactured by San-Ei Gen F.F.I., Inc.), 5 g of a yogurt flavoring agent (manufactured by San-Ei Gen F.F.I., Inc.), and 24 g of a glycerin fatty acid ester preparation (manufactured by Riken Vitamin Co., Ltd.) were homogeneously mixed to obtain a powder mixture. The powder mixture was continuously tableted using a tablet machine (manufactured by Hata Iron Works Co., Ltd.) at a tableting speed of 12 tablets/min and a pressure of 9.8 KPa to obtain tablets each having a weight of 0.5 g. As a result, 1800 tablets (about 900 g) containing lactoperoxidase were produced as an agent for relieving the symptoms of inflammatory bowel diseases and irritable bowel syndrome and/or treating inflammatory bowel diseases and irritable bowel syndrome.

### Example 3

10.8 kg of a whey protein hydrolysate (manufactured by Morinaga Milk Industry Co., Ltd.), 36 kg of dextrin (manufactured by Showa Sangyo Co., Ltd.), and small amounts of water-soluble vitamin and minerals were dissolved in 200 kg of water to prepare an aqueous phase in a tank. On the other hand, 3 kg of soybean cooking oil (manufactured by Taiyo-yushi Co., Ltd.), 8.5 kg of palm oil (manufactured by Taiyo-yushi Co., Ltd.), 2.5 kg of safflower oil (manufactured by Taiyo-yushi Co., Ltd.), 0.2 kg of lecithin (manufactured by AJINOMOTO CO., INC.), 0.2 kg of fatty acid monoglyceride (manufactured by KAO Corporation), and a small amount of oil-soluble vitamin were mixed and dissolved to prepare an oil phase. The oil phase was added to the aqueous phase contained in the tank, and then they were mixed by stirring, heated to 70°C, and homogenized by a homogenizer at a pressure of 14.7 MPa. Then, the mixture was sterilized at 90°C for 10 minutes, condensed, and spray-dried to prepare about 59 kg of an intermediate powder. To 50 kg of the intermediate powder, 6.8 kg of sucrose (manufactured by Hokuren), 167 g of an amino acid mixture powder (manufactured by AJINOMOTO CO., INC.), and 60 g of the lactoperoxidase obtained in the Example 1 were added, and they were homogeneously mixed to obtain about 56 kg of an enteral nutrition powder containing lactoperoxidase having the effect of preventing intestinal disorders and/or relieving the symptoms of intestinal disorders.

Hereinbelow, the present invention will be described in detail with reference to the following test examples.

### (Test Example 1)

The effect of lactoperoxidase on symptoms of colitis was determined by carrying out a test using animal models of intestinal disorders such as ulcerative colitis and Crohn's disease.

### (1) Preparation of Samples

The lactoperoxidase obtained in the Example 1 was dissolved in purified water so that the concentration of the lactoperoxidase was 12.5% by mass to prepare a test sample. On the other hand, bovine serum albumin (manufactured by Sigma) was dissolved in purified water so that the concentration of the bovine serum albumin was 12.5% by mass to prepare a control sample.

As test animals, 7-week-old CBA/J female mice (purchased from Charles River Laboratories Japan, Inc.) were prepared. Dextran sulfate sodium (molecular weight: 36,000 to 50,000, MP Biomedicals) was fed to the mice to induce colitis. These mice were used as animal colitis models.

### (2) Test Method

### a) Feeding of Sample and Measurement of Body Weight

32 seven-week-old CBA/J female mice were housed in a cage equipped with a net for preventing eating of feces, and grown by feeding Labo MR stock (manufactured by Nosan Corporation) and drinking water for 1 week for acclimation. Then, as shown in Table 1, two groups each containing 6 mice and two groups each containing 10 mice (4 groups in total) were prepared.

Dextran sulfate sodium was not fed to the mice of Group 1 and Group 2. To the mice of Group 1, 0.5 ml of the control sample was orally administered using a probe once a day for 10 days. To the mice of Group 2, 0.5 ml of the test sample was orally administered using a probe once a day for 10 days.

On the other hand, dextran sulfate sodium was fed to the mice belonging to colitis model groups (Group 3 and Group 4) to induce colitis. To the mice of Group 3, 0.5 ml of the control sample was orally administered using a probe once a day for 10 days, and at the same time, dextran sulfate sodium was also administered to the mice for 9 days from day 2 to day 10 after the initiation of administration of the control sample by feeding drinking water containing dextran sulfate sodium dissolved therein at a concentration of 3.0% by mass.

To the mice of Group 4, 0.5 ml of the test sample was orally administered using a probe once a day for 10 days, and at the same time, dextran sulfate sodium was also administered to the mice for 9 days from day 2 to day 10 after the initiation of administration of the test sample by feeding drinking water containing dextran sulfate sodium dissolved therein at a concentration of 3.0% by mass.

**Table 1**

| Test Group | Administration of Dextran Sulfate Sodium | Sample Administered | Number of Animals |
|---|---|---|---|
| Group 1 | Not Administered | Control Sample | 6 |
| Group 2 | Not Administered | Test Sample | 6 |
| Group 3 | Administered | Control Sample | 10 |
| Group 4 | Administered | Test Sample | 10 |

The body weight of each of the mice belonging to Groups 1 to 4 was measured on day 2 after the initiation of administration of the sample and on the final day of administration of the sample (i.e., on day 10). After the completion of administration of the sample, each of the mice belonging to Groups 1 to 4 was dissected under anesthesia to excise the large intestine.

### b) Measurement of Length of Large Intestine

After the completion of administration of the sample, each of the mice belonging to Groups 1 to 4 was dissected under anesthesia to excise the large intestine. The length of the excised large intestine of each of the mice was measured using a ruler to determine an average ± standard deviation as an indicator of hypertrophy of intestinal wall.

### c) Scoring of Colitis

The remaining large intestine excised in the above manner was twisted around a round bar, fastened to the round bar with pins, and fixed using 10% phosphate-buffered formalin (manufactured by Nacalai tesque). Then, the large intestine was embedded in paraffin in the usual manner, sliced, and stained with hematoxylin-eosin to make a preparation. The severity of colitis of the preparation was histopathologically scored according to the criteria shown in Table 2 based on a method developed by Cooper et al. (see, for example, Laboratory Investigation, vol. 69, pp. 238-249, 1993, USA). In addition, the ratio of a lesioned part of colitis to the entire large intestine was scored according to the criteria shown in Table 3. These scores were multiplied to determine a colitis score, and then the average ± standard deviation of the colitis score of each test group was determined.

### d) Statistical Analysis

The change in body weight, length of the large intestine, and colitis score were statistically analyzed among test groups by one-way analysis of variance and the Tukey-Kramer method to determine whether a significant difference existed between test groups at a significance level of less than 5% (p<0.05).

**Table 2**

| Score | Criteria (Colitis Severity) |
|---|---|
| 0 | Normal crypt structure |
| 1 | 1/3 of basal crypts are lost |
| 2 | 2/3 of basal crypts are lost |
| 3 | Epithelial cells in the surface layer of the mucous membrane remain but all the crypts are lost |
| 4 | Epithelial cells in the surface layer of the mucous membrane and all the crypts are lost (erosion) |

**Table 3**

| Score | Criteria (ratio of lesioned part of colitis to entire large intestine:%) |
|---|---|
| 0 | 0 |
| 1 | 1 to 25 |
| 2 | 26 to 50 |
| 3 | 51 to 75 |
| 4 | 76 to 100 |

### (3) Test Results

The test results are shown in Tables 4 to 6. Tables 4 to 6 show the average ± standard deviation of the amount of change in body weight of each test group (from day 2 to day 10 after the initiation of sample administration), the average ± standard deviation of the length of the large intestine of each test group, and the average ± standard deviation of the colitis score of each test group, respectively. As shown in Table 4, the body weights of the normal animal models belonging to Group 1 to which the control sample had been administered were increased by 0.85 g on average, whereas the body weights of the animal colitis models belonging to Group 3 to which the control sample had been administered were decreased by 1.73 g on average. Between Group 1 and Group 3, a significant difference was observed. On the other hand, the body weights of the animal colitis models belonging to Group 4 to which lactoperoxidase had been administered were decreased by only 0.30 g on average. From the result, it was found that body weight loss of the animal colitis models belonging to Group 4 was suppressed as compared to the animal colitis models belonging to Group 3 to which the control sample had been administered. Between Group 3 and Group 4, a significant difference was observed. It is to be noted that the normal animal models belonging to Group 2 to which lactoperoxidase had been administered gained weight almost similarly to the normal animal models belonging to Group 1 to which the control sample had been administered. Therefore, between Group 1 and Group 2, a significant difference was not observed.

As shown in Table 5, the average length of large intestine of the normal animal models belonging to Group 1 to which the control sample had been administered was 9.1 cm, whereas the average length of large intestine of the animal colitis models belonging to Group 3 to which the control sample had been administered was as short as 5.8 cm. Between Group 1 and Group 3, a significant difference was observed. On the other hand, the average length of large intestine of the animal colitis models belonging to Group 4 to which lactoperoxidase had been administered was 6.7 cm. From the result, it was found that colon shortening of the animal colitis models belonging to Group 4 was suppressed as compared to the animal colitis models belonging to Group 3 to which the control sample had been administered. Between Group 3 and Group 4, a significant difference was observed. It is to be noted that the large intestine lengths of the normal animal models belonging to Group 2 to which lactoperoxidase had been administered were substantially the same as those of the normal animal models belonging to Group 1 to which the control sample had been administered. Therefore, between Group 1 and Group 2, a significant difference was not observed.

As shown in Table 6, the average colitis score of the normal animal models belonging to Group 1 to which the control sample had been administered was 0, whereas the average colitis score of the animal colitis models belonging to Group 3 to which the control sample had been administered was as high as 8.0. Between Group 1 and Group 3, a significant difference was observed. On the other hand, the average colitis score of the animal colitis models belonging to Group 4 to which lactoperoxidase had been administered was 5.1. From the result, it was found that the colitis scores of the animal colitis models belonging to Group 4 were improved as compared to those of the animal colitis models belonging to Group 3 to which the control sample had been administered. Between Group 3 and Group 4, a significant difference was observed.

It is apparent from the above results that lactoperoxidase has the effect of improving the symptoms of colitis, such as body-weight loss and colon shortening, and the colitis score from the viewpoint of statistical significance.

**Table 4**

| Test Group | Change in Body Weight (g) |
|---|---|
| Group 1 | 0.85±0.72^{a•b} |
| Group 2 | 1.43±0.64^{a} |
| Group 3 | -1.73±1.31^{c} |
| Group 4 | -0.30±1.01^{b} |

| | |
|---|---|
| *) Analysis was performed by the Tukey-Kramer method to determine whether a significant difference existed between test groups. It is to be noted that between test groups not sharing a common alphabet, there is a significant difference at a significance level of less than 5% (p<0.05). | |

**Table 5**

| Test Group | Length of Large Intestine (cm) |
|---|---|
| Group 1 | 9.1±0.9^{a} |
| Group 2 | 9.0±0.5^{a•b} |
| Group 3 | 5.8±0.3^{c} |
| Group 4 | 6.7±0.5^{d} |

| | |
|---|---|
| *) Analysis was performed by the Tukey-Kramer method to determine whether a significant difference existed between test groups. It is to be noted that between test groups not sharing a common alphabet, there is a significant difference at a significance level of less than 5% (p<0.05). | |

**Table 6**

| Test Group | Colitis Score |
|---|---|
| Group 1 | 0.0±0.0^{a} |
| Group 2 | 0.0±0.0^{a•b} |
| Group 3 | 8.0±1.8^{c} |
| Group 4 | 5.1±1.4^{d} |

| | |
|---|---|
| *) Analysis was performed by the Tukey-Kramer method to determine whether a significant difference existed between test groups. It is to be noted that between test groups not sharing a common alphabet, there is a significant difference at a significance level of less than 5% (p<0.05). | |

### Industrial Applicability

The pharmaceutical composition, food or drink, or feed for intestinal disorders according to the present invention is highly safe for humans and animals, and therefore can be daily administered or ingested to relieve symptoms resulting from intestinal disorders such as ulcerative colitis and Crohn's disease (i.e., induction and maintenance of remission). In addition, lactoperoxidase to be used in the present invention as an active ingredient can be produced in large quantity from a raw material such as milk, and therefore it is possible to provide a pharmaceutical composition or a food or drink for intestinal disorders at low price.

## Claims

1. Lactoperoxidase for use in a method of prevention and/or treatment of an intestinal disorder, wherein the intestinal disorder is at least one or more selected from among ulcerative colitis and Crohn's disease.

2. A pharmaceutical composition comprising lactoperoxidase as an active ingredient and a pharmaceutically acceptable carrier for use in a method of prevention and/or treatment of an intestinal disorder, wherein the intestinal disorder is at least one or more selected from among ulcerative colitis and Crohn's disease.

3. A food, a drink, a food additive or a feed comprising lactoperoxidase as an active ingredient for use in a method of prevention and/or treatment of an intestinal disorder, wherein the intestinal disorder is at least one or more selected from among ulcerative colitis and Crohn's disease.

4. The food or drink for the use according to claim 3, which is provided as a health food, a functional food, a food for special dietary uses, a food with nutrient function claims, or a food for specified health uses.

## Patentansprüche

1. Lactoperoxidase zur Verwendung in einem Verfahren zur Prävention und/oder Behandlung einer intestinalen Störung, wobei die intestinale Störung wenigstens eine oder mehrere, ausgewählt aus Colitis ulcerosa und Crohn'scher Krankheit, ist.

2. Pharmazeutische Zusammensetzung, die Lactoperoxidase als aktives Ingrediens und einen pharmazeutisch annehmbaren Träger umfasst, zur Verwendung in einem Verfahren zur Prävention und/oder Behandlung einer intestinalen Störung, wobei die intestinale Störung wenigstens eine oder mehrere, ausgewählt aus Colitis ulcerosa und Crohn'scher Krankheit, ist.

3. Nahrungsmittel, Getränk, Nahrungsmittelzusatzstoff oder Futter, das Lactoperoxidase als aktives Ingrediens umfasst, zur Verwendung in einem Verfahren zur Prävention und/oder Behandlung einer intestinalen Störung, wobei die intestinale Störung wenigstens eine oder mehrere, ausgewählt aus Colitis ulcerosa und Crohn'scher Krankheit, ist.

4. Nahrungsmittel oder Getränk zur Verwendung gemäß Anspruch 3, das als Gesundheitsnahrungsmittel, als Functional Food, ein Nahrungsmittel für spezielle diätetische Verwendungen, ein Nahrungsmittel mit Nährstofffunktionsansprüchen oder Nahrungsmittel für spezifizierte Gesundheitsverwendungen bereitgestellt wird.

## Revendications

1. Lactopéroxydase pour l'utilisation dans un procédé de prévention et/ou de traitement d'un trouble intestinal, le trouble intestinal étant au moins ou plusieurs choisi(s) parmi la colite ulcéreuse et la maladie de Crohn.

2. Composition pharmaceutique comprenant la lactopéroxydase en tant que principe actif et un support pharmaceutiquement acceptable pour l'utilisation dans un procédé de prévention et/ou de traitement d'un trouble intestinal, le trouble intestinal étant au moins ou plusieurs choisi(s) parmi la colite ulcéreuse et la maladie de Crohn.

3. Aliment, boisson, additif alimentaire ou nourriture comprenant la lactopéroxydase en tant que principe actif pour l'utilisation dans un procédé de prévention et/ou de traitement d'un trouble intestinal, le trouble intestinal étant au moins ou plusieurs choisi(s) parmi la colite ulcéreuse et la maladie de Crohn.

4. Aliment ou boisson pour l'utilisation selon la revendication 3 qui est offerte comme un aliment de santé, un aliment fonctionnel, un aliment pour des utilisations spéciales diététiques, un aliment avec des allégations nutritionnelles fonctionnelles ou un aliment pour des utilisations spécifiées pour la santé.
